# EUROPEAN PATENT SPECIFICATION

(11) **EP 3 755 373 B1**
(45) Date of publication and mention of the grant of the patent: **28.07.2021**
(21) Application number: 18712544.8
(22) Date of filing: 15.03.2018
(51) Int. Cl.: A61K 47/26, A61K 9/08, A61K 31/137, A61K 31/135

(54) **ORAL SOLUTIONS COMPRISING TRAMADOL**
ORALE LÖSUNGEN MIT TRAMADOL
SOLUTIONS ORALES COMPRENANT DU TRAMADOL

(30) Priority: 20.02.2018 GR 20180100068
(43) Date of publication of application: 30.12.2020
(73) Proprietor: LABOMED PHARMACEUTICAL COMPANY S.A., 19441 Koropi, Attica (GR)
(72) Inventor: PANAGIOTOPOULOS, Tsampikos Dimitrios, 15124 Marousi (GR); TZIALA, Soultana, 19016 Artemida, Attika (GR)
(74) Representative: Roukounas, Dimitrios
(86) International application number: PCT/EP2018/056566
(87) International publication number: WO 2019/161938

(56) References cited:
- WO-A1-2012/049550
- WO-A1-2014/083071
- DE-A1-102006 056 458
- MX-A- PA03 012 048
- LIN T.-F. ET AL.: "Compatibility and stability of binary mixtures of ketorolac tromethamine and tramadol hydrochloride injection concentrate and diluted infusion solution", ACTA ANAESTHESIOLOGICA TAIWANICA, ELSEVIER, AMSTERDAM, NL, vol. 48, no. 3, 1 September 2010 (2010-09-01), pages 117-121, XP027306392, ISSN: 1875-4597 [retrieved on 2010-09-01]

## Description

### FIELD OF THE INVENTION

The present invention relates to oral pharmaceutical solutions comprising as active ingredient tramadol or a pharmaceutically acceptable salt thereof.

### BACKGROUND OF THE INVENTION

Tramadol ((1RS,2RS)-2-(dimethylaminomethyl)-1-(3-methoxyphenyl)cyclohexanol) is a well-established active pharmaceutical ingredient disclosed in US 3,652,589, which is used as a non-narcotic analgesic medicament. Tramadol is mainly used in the form of its hydrochloride salt.

Tramadol hydrochloride was launched and marketed as "Tramal®" by the German pharmaceutical company Grünenthal GmbH in 1977 in West Germany, and 20 years later it was launched in countries such as the UK, US, and Australia. The currently commercially available "Tramal® oral drops, solution", contains 100 mg tramadol hydrochloride per 1 ml of solution. Tramal® also contains as excipients potassium sorbate, glycerol, propylene glycol, 200 mg/ml sucrose, sodium cyclamate, saccharin sodium, 1 mg/ml polyoxyl 40 hydrogenated castor oil, mint oil, aniseed flavour and purified water. Tramal® is packaged in a dropper container, consisting of a glass bottle with inserted plastic dropper container or in a container with dispenser pump.

Tramadol hydrochloride is also marketed under many brand names worldwide. Another example of commercially available oral liquid product is "Tramadol 100 mg/ml oral drops, solution" which is marketed in several European countries, and contains 100 mg tramadol hydrochloride per 1 ml of solution. This product also contains as excipients 200 mg/ml sucrose, saccharin sodium, potassium sorbate, polysorbate 20, aniseed oil, peppermint oil, hydrochloric acid (for pH adjustment) and purified water. This product is packaged in a dropper container, consisting of a glass bottle with inserted plastic dropper applicator.

According, to the Patient Information Leaflet (PIL) of marketed tramadol hydrochloride oral liquid products, the solutions are administered in the form of oral drops. Importantly, the drops should be diluted with water before administration. The usual dose for adults and children aged 12 and over is 50 mg to 100 mg tramadol (20 to 40 drops), three to four times per day. The maximum allowed dose of Tramadol oral drops is generally 400 mg (160 drops) per day. For acute pain, a starting dose of 100 mg is generally required since the effect begins later than with other pain relievers.

However, according to the "Guideline on pharmaceutical development of medicines for paediatric use" of the European Medicines Agency, oral drops can provide a useful means to administer medicinal products only in low doses or small volumes. Importantly, according to the guideline there is a risk of counting the incorrect number of drops, and the accuracy and precision of the volume administered should always be justified in relation to the criticality of the dose. In order to avoid counting errors, alternative measuring devices should be considered where the dose comprises more than 10 drops.

Apparently, this lack of convenience due to excessive number of drops required to deliver the extremely high doses of tramadol, as well as due to the rather inconvenient reconstitution procedure, may result in high incidence of non-compliance and ineffective therapy.

Thus, as a means of both ensuring flexibility in dose titration and advanced patient compliance, the development of oral pharmaceutical solutions which may also be administered by means of a device suitable for accurately measuring the prescribed volume, such as an oral syringe, a spoon or a cup, and which may also be administered without first diluting them with a liquid carrier, is definitely an existing need.

However, it is extremely challenging to formulate tolerable tramadol oral solutions that can be administered without first diluting them with a liquid carrier, since it is considered difficult to effectively taste mask the bad and very bitter taste of tramadol and its pharmaceutically acceptable salts, even at low concentrations.

According, for example, to US 6,723,343 despite the excellent efficacy of tramadol in pain control, the active ingredient tramadol and its readily soluble salts have an intensely bitter taste. As a consequence, available formulations of tramadol are poorly accepted and patients fail to observe the dosage instructions.

In these cases, the prior art indicates that high concentrations of taste masking agents such as sucrose, sugar alcohols, essential oils and sweeteners are necessary for masking the bitterness of the active ingredient. But even such high quantities are not proved sufficient to provide an effectively taste masked formulation. Thus, tramadol or its pharmaceutically acceptable salts are inevitably formulated in the form of oral drops that should be first diluted with a liquid carrier, such as water, prior to their administration.

Moreover, general health-consciousness and orientation to low calorie diet are making consumers avoid sweeteners and especially sugars which has become perhaps the most discussed dietary issue in recent years with more and more research studies linking the effects of sugars to greater risks of, among others, obesity, type 2 diabetes and decayed teeth. Thus, it would be desirable to arrive at low calorie formulations, which would be suitable for use by, for example, pediatric patients and diabetic patients

The present invention addresses the problems of the prior art knowledge by advantageously providing an oral pharmaceutical solution which comprises tramadol or a pharmaceutically acceptable salt thereof and which can be administered without first diluting it with a liquid carrier.

### SUMMARY OF INVENTION

The present invention provides an oral pharmaceutical solution comprising as active ingredient tramadol or a pharmaceutically acceptable salt thereof.

The oral pharmaceutical solution according to the invention comprises tramadol or a pharmaceutically acceptable salt thereof in a concentration of 10 mg/ml to 40 mg/ml and a pharmaceutically acceptable liquid carrier.

Preferably, the oral pharmaceutical solution according to the invention comprises from 10 mg/ml to 40 mg/ml tramadol or a pharmaceutically acceptable salt thereof and one or more polyols, wherein the total concentration of polyols is from 5 mg/ml to 200 mg/ml.

The oral pharmaceutical solution according to the invention presents excellent taste masking effect while at the same time allows the optimal selection of concentration of taste masking excipients, surfactants or cosolvents at the lowest feasible level. The use of these excipients at the lowest feasible level is very important, particularly in the case of formulations intended for paediatric population.

The oral pharmaceutical solution according to the invention provides, for the administration of a specific dose, a higher and more consistent palatability effect than currently available liquid dosage forms of tramadol.

The oral pharmaceutical solution according to the invention does not have to be first diluted with a liquid carrier, prior to its administration.

### DETAILED DESCRIPTION OF THE INVENTION

The present invention provides an oral pharmaceutical solution comprising tramadol or a pharmaceutically acceptable salt thereof, in association with a pharmaceutically acceptable liquid carrier.

As used throughout this description and claims, the term "sugar" refers to soluble saccharides, having sweet taste. Examples of sugars include, but are not limited to sucrose, glucose, dextrose, fructose and galactose.

As used throughout this description and claims, the term "polyol" (polyhydric alcohol) refers to pharmaceutical excipients containing multiple hydroxyl groups, which however are not sugars. Polyols are used as sweeteners and bulking agents. They occur naturally in foods and come from plant products such as fruits and berries. Typical examples of suitable polyols according to the invention are sugar alcohols such as, glycerol, maltitol, sorbitol, xylitol, erythritol, isomalt and lactitol as well as propylene glycol and polyvinyl alcohol. Preferably, the polyol is glycerol, maltitol, sorbitol or xylitol. More preferably, the polyol is maltitol or xylitol.

The term "mg/ml" refers to mg of the respective ingredient per 1 ml of the oral solution.

In the prior art, attempts have been made to mitigate or reduce the bad and very bitter taste and aftertaste imparted by tramadol and its pharmaceutically acceptable salts when taken in the form of an oral liquid. These attempts include use of taste masking agents such as sucrose, sugar alcohols, essential oils and sweeteners in relatively high concentrations or reformulation of tramadol or its pharmaceutically acceptable salts in extremely low concentrations.

The inefficient use of high quantities of taste masking agents in the oral liquid formulations described in the prior art, proved not only insufficient to effectively taste mask the bitter taste and aftertaste of tramadol and its pharmaceutically acceptable salts so that to be feasible for these oral liquids to be administered without diluting them with a liquid carrier prior to their administration, but also proved to overwhelm bitterness with an unpleasant sweetness.

Now, it has been unexpectedly found that extremely low concentrations of tramadol or its pharmaceutically acceptable salts cannot be administered in the form of an oral solution without first diluting it with a liquid carrier. It is assumed that the bigger volumes of solution required in this case to be administered directly to mouth give rise to a bitterer aftertaste that lasts longer, since some of the bitter tasting tramadol or pharmaceutically acceptable salts thereof is retained in the mouth long enough to contact the taste buds of the tongue. In this case, the relatively high concentrations of taste masking agents that are necessary for masking the bitterness of tramadol or its pharmaceutically acceptable salts cause a negative effect on solubility, resulting in the formation of a suspension, with the presence of visible particles that become apparent upon production.

Hence, disclosed herein is the unexpected finding that, for the administration of a specific dose, oral solutions in a certain concentration range of tramadol or its pharmaceutically acceptable salts thereof provide an improved palatability effect compared to solutions comprising tramadol or pharmaceutically acceptable salts thereof outside this concentration range. This unexpected finding allows on the one hand the use of lower concentrations of excipients, including taste masking agents, and on the other hand allows the administration of the solutions without first diluting them with a liquid carrier.

The oral pharmaceutical solution according to the invention comprises tramadol or a pharmaceutically acceptable salt thereof in a concentration of 10 mg/ml to 40 mg/ml and a pharmaceutically acceptable liquid carrier.

Preferably, the oral pharmaceutical solution according to the invention comprises one or more polyols, wherein the total concentration of polyols is from 5 mg/ml to 200 mg/ml. More preferably, the oral pharmaceutical solution according to the invention comprises one or more polyols, wherein the total concentration of polyols is from 20 mg/ml to 150 mg/ml.

Preferably, the oral pharmaceutical solution according to the invention comprises from 20 mg/ml to 30 mg/ml of tramadol or a pharmaceutically acceptable salt thereof.

Preferably, the oral pharmaceutical solution according to the invention comprises one or more polyols, wherein the total concentration of polyols is from 20 mg/ml to 150 mg/ml.

Preferably, the oral pharmaceutical solution according to the invention comprises as active ingredient tramadol hydrochloride.

Preferably, the oral pharmaceutical solution according to the invention comprises tramadol hydrochloride as the only active ingredient.

Preferably, the oral pharmaceutical solution according to the invention optionally comprises one or more sugars, wherein the total concentration of sugars is lower than 50 mg/ml. More preferably, the oral pharmaceutical solution according to the invention is free of sugars.

Lower concentrations or even absence of sugars and essential oils relieve the oral solutions of the "heavy" oily texture, disagreeable "syrupy" mouthfeel which is prominent in the solutions described in the prior art. It is assumed that lower concentrations or even absence of these taste-masking excipients, which increase the viscosity of the solution, helps bitter tasting tramadol or its pharmaceutically acceptable salts to be retained less time in the mouth.

Thus, in a preferred embodiment, the oral pharmaceutical solution according to the invention is also free of essential oils.

The oral pharmaceutical solution according to the invention presents excellent taste-masking effect while at the same time allows the optimal selection of concentration of taste-masking excipients at the lowest feasible level. Hence, use of non-ionic surfactants or co-solvents is not deemed necessary in order to overcome solubility problems caused by high concentrations of taste-masking excipients.

Thus, in a preferred embodiment, the oral pharmaceutical solution according to the invention is also free of surfactants and cosolvents such as castor oil, modified castor oils, ethanol, polyethylene glycol, polyvinylpyrrolidone, copolyvidone and sorbitan monolaurate.

Preferably, the pharmaceutically acceptable liquid carrier according to the invention comprises water.

The oral pharmaceutical solution of tramadol or its pharmaceutically acceptable salt, according to the invention may also comprise additional excipients commonly used in preparing oral liquid compositions, such as antimicrobial preservatives, antioxidants, buffering agents, sweeteners and flavouring agents.

Antimicrobial preservatives may include but are not limited to sodium benzoate, benzoic acid, boric acid, sorbic acid and their salts thereof, benzyl alcohol, parahydroxybenzoic acids and their alkyl esters, methyl, ethyl and propyl parahydroxybenzoates and their salts or mixtures thereof.

Antioxidants which may be used in the present invention comprise, amongst others, butylated hydroxytoluene, butylated hydroxyanisole, ethylenediamine tetraacetic acid ("EDTA"), ascorbic acid, sodium metabisulfite and propyl gallate or any combinations thereof.

Buffering agents may include but are not limited to ascorbic acid, acetic acid, tartaric acid, citric acid monohydrate, sodium citrate, potassium citrate, sodium phosphate, calcium carbonate, sodium bicarbonate, calcium phosphate, carbonated calcium phosphate, magnesium hydroxide or mixtures thereof.

Sweeteners may include but are not limited to aspartame, acesulfame potassium, thaumatin, saccharin and salts thereof, sodium cyclamate, glycyrrhizin, monosodium glycyrrhizinate, monoamonium glycyrrhizinate or mixtures thereof.

Flavouring agents may include but are not limited to fruit flavours such as orange, banana, strawberry, cherry, wild cherry, lemon and the like and other flavourings, such as cardamom, anise, mint, menthol, vanillin, bubble gum or mixtures thereof.

The oral pharmaceutical solution according to the invention is supplied as multidose preparation. Each dose from a multidose container may be administered by means of a device suitable for accurately measuring the prescribed volume. The device is usually a spoon or a cup for volumes of 5 mL or multiples thereof, or an oral syringe for other volumes. Preferably, the device is an oral syringe.

In a preferred embodiment, the present invention provides an oral solution comprising tramadol or a pharmaceutically acceptable salt thereof for use in a method of treatment wherein the solution is orally administered without first diluting it with a liquid carrier.

The oral pharmaceutical solution of the present invention may be prepared using methods well known in the prior art and using regular manufacturing equipment. For example, it may be prepared using the following process:
The active ingredient and the excipients are weighed. Purified water is added into a vessel. A polyol and the active ingredient are successively dissolved into purified water under stirring. The remaining excipients are successively added under continuous stirring, until complete dissolution. Finally, the volume is adjusted with purified water.

The final solution is optionally filtered over a 10 µm filter, and filled preferably in light-protective containers, such as amber type III glass 50 or 100 ml bottles sealed with child resistant, tamper evident screw caps.

### EXAMPLES

### EXAMPLE 1

The purpose of this experiment was to evaluate the palatability and acceptability of compositions similar to commercial "Tramal® oral drops, solution" product (i.e. composition A1) versus compositions with different active ingredient concentrations.

The quantitative composition of commercial "Tramal® oral drops, solution" product was approached by a set of laboratory studies, including inter alia HPLC measurements for assessing the concentration of propylene glycol, potassium sorbate and glycerol as well as organoleptic tests for the assessment of the concentration of the remaining flavouring and sweetening agents. Information regarding concentration of sucrose and polyoxyl 40 hydrogenated castor oil is publically available through the SmPC of the product.

The tramadol hydrochloride solutions were then prepared in the following manner: Purified water and propylene glycol were added into a vessel under continuous stirring, until complete dissolution. Tramadol hydrochloride, glycerol and polyoxyl 40 hydrogenated castor oil were successively added into the vessel under continuous stirring, until complete dissolution. Sodium saccharin, sodium cyclamate, sucrose, potassium sorbate, mint oil and the flavour were also successively added under continuous stirring, until complete dissolution. Finally, the volume was adjusted with purified water. The final solution was filled in glass 50 ml bottles.

**Table 1**

| **Composition** | **A1** | **A2** | **A3** |
|---|---|---|---|
| **Active ingredient** | **mg/ml** | | |
| Tramadol hydrochloride | 100 | 20 | 8 |

| **Excipients** | **mg/ml** | | |
|---|---|---|---|
| Potassium sorbate | 1.0 | | |
| Glycerol | 120 | | |
| Propylene glycol | 150 | | |
| Sucrose | 200 | | |
| Sodium cyclamate | 5.0 | | |
| Sodium saccharin | 5.0 | | |
| Polyoxyl 40 hydrogenated castor oil | 1.0 | | |
| Mint oil | 1.0 | | |
| Aniseed flavour | 1.0 | | |
| Purified water | q.s. to 1ml | | |

The solutions were studied in relation to their basic taste and aftertaste after the administration of 100 mg tramadol hydrochloride. The flavor test results were based on the analysis of five trained judges who are experienced in detailed flavor analysis. All solutions were administered by means of a cup and they were administered directly into mouth and swallowed i.e. without first diluting them with a liquid carrier prior to their administration.

**Table 2: Volume administered**

| | Composition A1 (100 mg/ml) | Composition A2 (20 mg/ml) | Composition A3 (8 mg/ml) |
|---|---|---|---|
| Volume administered | ***1 ml*** | ***5 m**l* | ***12.5 m**l* |

Five out of five judges preferred composition A2 to composition A1 since they all noticed that the bitter aftertaste was more intense in case of A1. Thus, composition A2 proved to provide, for the administration of the same dose, a higher and more consistent palatability effect than commercially available product of tramadol hydrochloride.

Moreover, five out of five judges also preferred composition A2 to composition A3 since they all noticed that the intense bitter aftertaste lasts longer in case of A3 probably due to bigger volume of solution required to be administered directly to mouth. Thus, it has been surprisingly found that composition A2 provides, for the administration of the same dose, a higher and more consistent palatability effect than solutions comprising higher or lower concentrations of tramadol hydrochloride.

It is assumed that bigger volumes give rise to a bitter aftertaste since some of the bitter tasting active ingredient is retained in the mouth long enough to contact the taste buds of the tongue. A liquid carrier comprising high concentrations of taste masking agents such as sucrose, sugar alcohols, essential oils and sweeteners seems to magnify this unacceptable aftertaste effect.

### EXAMPLE 2

The purpose of this experiment was to evaluate the palatability and acceptability of compositions similar to commercial "Tramadol oral drops, solution" product (i.e. composition A1') versus compositions with different active ingredient concentrations.

The quantitative composition of commercial "Tramadol oral drops, solution" product was approached by a set of laboratory studies, including inter alia an HPLC measurement for assessing the concentration of potassium sorbate as well as organoleptic tests for the assessment of the concentration of the remaining flavouring and sweetening agents. Information regarding concentration of sucrose is publically available through the SmPC of the product.

The tramadol hydrochloride solutions were then prepared in the following manner: Purified water and tramadol hydrochloride were added into a vessel under continuous stirring, until complete dissolution. The remaining excipients were also successively added under continuous stirring, until complete dissolution. Finally, the volume was adjusted with purified water. The final solution was filled in glass 50 ml bottles.

**Table 3**

| **Composition** | **A1'** | **A2'** | **A3'** |
|---|---|---|---|
| **Active ingredient** | **mg/ml** | | |
| Tramadol hydrochloride | 100 | 20 | 8 |

| **Excipients** | **mg/ml** | | |
|---|---|---|---|
| Potassium sorbate | 1.0 | | |
| Sucrose | 200 | | |
| Sodium saccharin | 5.0 | | |
| Polysorbate 20 | 1.0 | | |
| Peppermint oil | 2.0 | | |
| Aniseed oil | 2.0 | | |
| Purified water | q.s. to 1ml | | |

The solutions were studied in relation to their basic taste and aftertaste after the administration of 100 mg tramadol hydrochloride. The flavor test results were based on the analysis of five trained judges who are experienced in detailed flavor analysis. All solutions were administered by means of a cup and they were administered directly into mouth and swallowed i.e. without first diluting them with a liquid carrier prior to their administration.

**Table 4: Volume administered**

| | Composition A1' (100 mg/ml) | Composition A2' (20 mg/ml) | Composition A3' (8 mg/ml) |
|---|---|---|---|
| Volume administered | ***1 ml*** | ***5 m**l* | ***12.5 m**l* |

Five out of five judges preferred composition A2' to composition A1' since they all noticed that the bitter aftertaste was more intense in case of A1'. Thus, composition A2' proved to provide, for the administration of the same dose, a higher and more consistent palatability effect than commercially available product of tramadol hydrochloride.

Moreover, five out of five judges also preferred composition A2' to composition A3' since they all noticed that the intense bitter aftertaste lasts longer in case of A3' probably due to bigger volume of solution required to be administered directly to mouth. Thus, it has been surprisingly found that composition A2' provides, for the administration of the same dose, a higher and more consistent palatability effect than solutions comprising higher or lower concentrations of tramadol hydrochloride.

It is believed that bigger volumes give rise to a bitter aftertaste since some of the bitter tasting active ingredient is retained in the mouth long enough to contact the taste buds of the tongue. A liquid carrier comprising high concentrations of taste masking agents such as sucrose, essential oils and sweeteners seems to magnify this unacceptable aftertaste effect.

### EXAMPLE 3

The purpose of this experiment was to evaluate the solubility and palatability of compositions with relatively high concentrations of taste masking excipients but without use of surfactants or co-solvents.

The tramadol hydrochloride compositions were prepared in the following manner: Purified water was added into a vessel. Tramadol hydrochloride, glycerol and potassium sorbate were successively added into the vessel under continuous stirring, until complete dissolution. The remaining excipients were also successively added under continuous stirring, until complete dissolution. Finally, the volume was adjusted with purified water. The final solution was filled in glass 50 ml bottles.

**Table 5**

| **Composition** | **B1** | **B2** |
|---|---|---|
| **Active ingredient** | **mg/ml** | |
| Tramadol hydrochloride | 8 | 8 |

| **Excipients** | **mg/ml** | |
|---|---|---|
| Potassium sorbate | 1.0 | 1.0 |
| Glycerol | 200 | 200 |
| Sorbitol | 75 | 100 |
| Peppermint oil | 5.0 | - |
| Sodium cyclamate | 2.5 | 2.5 |
| Sodium saccharin | 2.5 | 2.5 |
| Aniseed flavour | 2.0 | 2.0 |
| Purified water | q.s. to 1ml | |

The descriptive analysis methodology adopted in this case is characterized in the following Table 6. The test results are illustrated in the following Table 7.

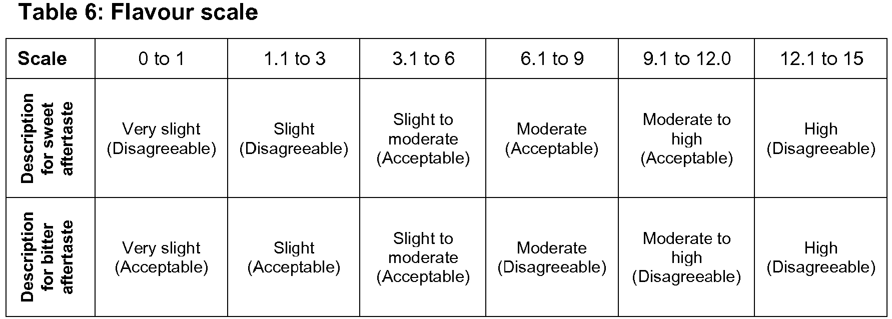

It is mentioned that, although a sweet aftertaste intensity lower than 12.0 is considered acceptable, when it is greater than 12.0, a rather disagreeable "syrupy" mouthfeel is revealed.

**Table 7**

| | | Composition B1 (8 mg/ml) | Composition B2 (8 mg/ml) |
|---|---|---|---|
| ***Volume administered*** | | ***12.5 m**l* | ***12.5 m**l* |
| | | | |
| Appearance of solution | | *Cloud, precipitation* | *Cloud, precipitation* |
| Aftertaste intensity after administered directly into mouth, without first diluting with a liquid carrier | Bitter | 5.7 | 6.8 |
| | Sweet | 12.5 | *13.1* |

The results of Table 7 indicate that the relatively high concentrations of taste masking agents that are necessary for masking the bitterness of tramadol hydrochloride even at low concentration of 8 mg/ml cause a negative effect on solubility, resulting in the formation of a suspension, with the presence of visible particles that become apparent upon production. Furthermore, both compositions of Table 7 exhibit a disagreeable sweet aftertaste.

### EXAMPLE 4

These tramadol hydrochloride oral liquids were prepared in the following manner: Purified water was added into a vessel. Glycerol and propylene glycol were added into the vessel under continuous stirring, until complete dissolution. Tramadol hydrochloride and polyoxyl 40 hydrogenated castor oil (if present), were successively added into the vessel under continuous stirring. Sodium saccharin, sodium cyclamate, potassium sorbate and the flavour were successively added under continuous stirring, until complete dissolution. Finally, the volume was adjusted with purified water.

**Table 8**

| **Composition** | **C1** | **C2** | **D1** | **D2** |
|---|---|---|---|---|
| **Active substance** | **mg/ml** | | | |
| Tramadol hydrochloride | 20 | 8 | 20 | 8 |

| **Excipients** | **mg/ml** | | | |
|---|---|---|---|---|
| Potassium sorbate | 1.0 | 1.0 | 1.0 | 1.0 |
| Glycerol | 100 | 100 | 75 | 200 |
| Propylene glycol | 25 | 25 | 25 | 25 |
| Sodium cyclamate | 2.5 | 2.5 | 1.0 | 2.5 |
| Sodium saccharin | 2.5 | 2.5 | 1.0 | 2.5 |
| Polyoxyl 40 hydrogenated castor oil | 1.0 | 1.0 | - | - |
| Aniseed flavour | 1.0 | 1.0 | 0.25 | 1.0 |
| Purified water | q.s. to 1 ml | | q.s. to 1ml | |

The compositions were studied in relation to their aftertaste after the administration of 100mg tramadol hydrochloride. The flavor test results were based on the analysis of five trained judges who are experienced in detailed flavor analysis.

The descriptive analysis methodology adopted is characterized in Table 6 of Example 3. The test results are illustrated in the following Tables 9a and 9b.

**Table 9a**

| | | Composition C1 (20 mg/ml) | Composition C2 (8 mg/ml) |
|---|---|---|---|
| **Volume administered** | | ***5 ml*** | ***12.5 ml*** |
| | | | |
| Appearance of solution | | *Clear solution* | *Clear solution* |
| Aftertaste intensity after administered directly into mouth, without first diluting with a liquid carrier | Bitter | 4.8 | 7.9 |
| | Sweet | 11.7 | 12.8 |

**Table 9b**

| | | Composition D1 (20 mg/ml) | Composition D2 (8 mg/ml) |
|---|---|---|---|
| ***Volume administered*** | | ***5 ml*** | ***12.5 ml*** |
| | | | |

| Appearance of solution | | *Clear solution* | *Cloud, precipitation* |
|---|---|---|---|
| Aftertaste intensity after administered directly into mouth, without first diluting with a liquid carrier | Bitter | 4.3 | 6.9 |
| | Sweet | *10.5* | 12.2 |

Interestingly, when the concentration of tramadol hydrochloride is as low as 8 mg/ml, a solution volume of 12.5 ml is required to be administered directly to mouth which gives rise to a bitter aftertaste since some of the bitter tasting active ingredient is retained in the mouth long enough to contact the taste buds of the tongue.

The results of table 9b indicate that the relatively high concentrations of taste masking agents that are necessary for masking the bitterness of tramadol hydrochloride at concentration of 8 mg/ml cause a negative effect on solubility, resulting in the formation of a suspension, with the presence of visible particles that become apparent upon production. Hence, use of non-ionic surfactants or co-solvents is deemed necessary in order to overcome solubility problems.

On the other hand, tramadol hydrochloride compositions provide an acceptable result at the concentration of 20 mg/ml of tramadol hydrochloride. At this concentration level the compositions present excellent taste masking effect while at the same time the optimal selection of concentration of taste masking excipients at the lowest feasible level is allowed.

### EXAMPLE 5

Table 10 shows preferred oral solution compositions according to the present invention.

These tramadol hydrochloride compositions were prepared in the following manner: Purified water was added into a vessel. Maltitol, xylitol, propylene glycol (if present) and sucrose (if present) were added into the vessel under continuous stirring, until complete dissolution. Tramadol hydrochloride, was added into the vessel under continuous stirring. Sodium saccharin, sodium methyl parahydroxybenzoate and the flavour were successively added under continuous stirring, until complete dissolution. Finally the volume was adjusted with purified water.

**Table 10**

| | **Composition** | | | |
|---|---|---|---|---|
| | **I** | **II** | **III** | **IV** |
| **Active ingredient** | **mg/ml** | | | |
| Tramadol hydrochloride | 16 | 25 | 20 | 25 |

| **Excipients** | **mg/ml** | | | |
|---|---|---|---|---|
| Maltitol | 20 | 25 | 15 | 15 |
| Xylitol | 20 | 25 | 15 | 15 |
| Sucrose | - | - | - | 15 |
| Propylene glycol | - | 25 | 25 | 25 |
| Sodium saccharin | 0.5 | 0.5 | 1.0 | 0.5 |
| Sodium methyl parahydroxybenzoate | 0.5 | 0.5 | 0.5 | 0.5 |
| Raspberry flavour | 0.5 | 1.0 | 1.0 | 1.0 |
| Purified water | q.s.to 1 ml | q.s.to 1 ml | q.s. to 1 ml | q.s. to 1 ml |

The solutions were studied in relation to their aftertaste after the administration of 100 mg tramadol hydrochloride. The flavor test results were based on the analysis of five trained judges who are experienced in detailed flavor analysis. All solutions were administered by means of an oral syringe with 0.25 ml increments and they were administered directly into mouth and swallowed, without first diluting them with a liquid carrier prior to their administration.

The descriptive analysis methodology adopted is characterized in Table 6 of Example 3. The test results are illustrated in the following Table 11.

**Table 11**

| | | **Composition** | | | |
|---|---|---|---|---|---|
| | | **I** | **II** | **III** | **IV** |
| **Volume administered** | | **6.25 ml** | **4 ml** | **5 ml** | **4 ml** |
| Aftertaste intensity after administered directly into mouth, without first diluting with a liquid carrier | Bitter | *3.1* | *3.6* | *3.4* | *3.4* |
| | Sweet | *10.7* | *9.4* | *10.2* | *11.4* |

## Claims

1. Oral pharmaceutical solution comprising from 10 mg/ml to 40 mg/ml tramadol hydrochloride as the only active ingredient and a pharmaceutically acceptable liquid carrier, wherein the solution comprises one or more polyols, wherein the total concentration of polyols is from 5 mg/ml to 200 mg/ml and wherein the solution is free of sugars.

2. Oral pharmaceutical solution according to claim 1, wherein the total concentration of polyols is from 20 mg/ml to 150 mg/ml.

3. Oral pharmaceutical solution according to claim 1 or 2, wherein the one or more polyols are selected from sugar alcohols, propylene glycol and polyvinyl alcohol.

4. Oral pharmaceutical solution according to any one of claims 1 to 3, wherein the one or more polyols are selected from glycerol, maltitol, sorbitol, xylitol, erythritol, isomalt and lactitol.

5. Oral pharmaceutical solution according to any one of claims 1 or 4, wherein the one or more polyols are selected from glycerol, maltitol, sorbitol and xylitol.

6. Oral pharmaceutical solution according to any one of claims 1 to 5, wherein the one or more polyols are selected from maltitol and xylitol.

7. Oral pharmaceutical solution according to any one of claims 1 to 6 wherein the concentration of tramadol hydrochloride is from 20 mg/ml to 30 mg/ml.

8. Oral pharmaceutical solution according to any one of claims 1 to 7, wherein the sugars are selected from sucrose, glucose, dextrose, fructose and galactose.

9. Oral pharmaceutical solution according to any one of claims 1 to 8, wherein the solution is free of essential oils, castor oil, modified castor oils, ethanol, polyethylene glycol, polyvinylpyrrolidone, copolyvidone and sorbitan monolaurate.

10. Oral pharmaceutical solution according to any one of claims 1 to 9, wherein the pharmaceutically acceptable liquid carrier comprises water.

11. Oral pharmaceutical solution according to any of claims 1 to 10, for use in a method of treatment wherein the solution is orally administered without first diluting it with a liquid carrier.

## Patentansprüche

1. Orale pharmazeutische Lösung, umfassend von 10 mg/ml bis 40 mg/ml Tramadolhydrochlorid als einziger Wirkstoff und einen pharmazeutisch akzeptablen flüssigen Trägerstoff, wobei die Lösung ein oder mehrere Polyol(e) umfasst, wobei die Gesamtkonzentration der Polyole von 5 mg/ml bis 200 mg/ml beträgt und wobei die Lösung frei von Zuckern ist.

2. Orale pharmazeutische Lösung nach Anspruch 1, wobei die Gesamtkonzentration der Polyole von 20 mg/ml bis 150 mg/ml beträgt.

3. Orale pharmazeutische Lösung nach Anspruch 1 oder 2, wobei das eine oder die mehreren Polyol(e) aus Zuckeralkoholen, Propylenglykol und Polyvinylalkohol ausgewählt ist/sind.

4. Orale pharmazeutische Lösung nach einem der Ansprüche 1 bis 3, wobei das eine oder die mehreren Polyol(e) aus Glycerin, Maltit, Sorbit, Xylit, Erythrit, Isomalt und Lactitol ausgewählt ist/sind.

5. Orale pharmazeutische Lösung nach einem der Ansprüche 1 bis 4, wobei das eine oder die mehreren Polyol(e) aus Glycerin, Maltit, Sorbit und Xylit ausgewählt ist/sind.

6. Orale pharmazeutische Lösung nach einem der Ansprüche 1 bis 5, wobei das eine oder die mehreren Polyol(e) aus Maltit und Xylit ausgewählt ist/sind.

7. Orale pharmazeutische Lösung nach einem der Ansprüche 1 bis 6, wobei die Konzentration von Tramadolhydrochlorid von 20 mg/ml bis 30 mg/ml beträgt.

8. Orale pharmazeutische Lösung nach einem der Ansprüche 1 bis 7, wobei die Zucker aus Saccharose, Glucose, Dextrose, Fructose und Galactose ausgewählt sind.

9. Orale pharmazeutische Lösung nach einem der Ansprüche 1 bis 8, wobei die Lösung frei von ätherischen Ölen, Rizinusöl, modifizierten Rizinusölen, Ethanol, Polyethylenglykol, Polyvinylpyrrolidon, Copolyvidon und Sorbitanmonolaurat ist.

10. Orale pharmazeutische Lösung nach einem der Ansprüche 1 bis 9, wobei der pharmazeutisch akzeptable flüssige Trägerstoff Wasser umfasst.

11. Orale pharmazeutische Lösung nach einem der Ansprüche 1 bis 10 zur Verwendung in einem Behandlungsverfahren, wobei die Lösung ohne vorheriges Verdünnen mit einem flüssigen Trägerstoff oral verabreicht wird.

## Revendications

1. Solution pharmaceutique orale comprenant de 10mg/ml à 40 mg/ml de chlorhydrate de tramadol en tant que l'unique principe actif et un vecteur liquide pharmaceutiquement acceptable, dans laquelle la solution comprend un ou plusieurs polyols, dans laquelle la concentration totale en polyols est de 5 mg/ml à 200 mg/ml et dans laquelle la solution est exempte de sucres.

2. Solution pharmaceutique orale selon la revendication 1, dans laquelle la concentration totale en polyols est de 20 mg/ml à 150 mg/ml.

3. Solution pharmaceutique orale selon la revendication 1 ou 2, dans laquelle les un ou plusieurs polyols sont sélectionnés parmi les alcools de sucre, le propylène glycol et l'alcool polyvinylique.

4. Solution pharmaceutique orale selon l'une quelconque des revendications 1 à 3, dans laquelle les un ou plusieurs polyols sont sélectionnés parmi le glycérol, le maltitol, le sorbitol, le xylitol, l'érythritol, l'isomalt et le lactitol.

5. Solution pharmaceutique orale selon l'une quelconque des revendications 1 à 4, dans laquelle les un ou plusieurs polyols sont sélectionnés parmi le glycérol, le maltitol, le sorbitol et le xylitol.

6. Solution pharmaceutique orale selon l'une quelconque des revendications 1 à 5, dans laquelle les un ou plusieurs polyols sont sélectionnés parmi le maltitol et le xylitol.

7. Solution pharmaceutique orale selon l'une quelconque des revendications 1 à 6 dans laquelle la concentration en chlorhydrate de tramadol est de 20 mg/ml à 30 mg/ml.

8. Solution pharmaceutique orale selon l'une quelconque des revendications 1 à 7, dans laquelle les sucres sont sélectionnés parmi le sucrose, le glucose, le dextrose, le fructose et le galactose.

9. Solution pharmaceutique orale selon l'une quelconque des revendications 1 à 8, dans laquelle la solution est dépourvue d'huiles essentielles, d'huile de ricin, d'huile de ricin modifiée, d'éthanol, de polyéthylène glycol, de polyvinylpyrrolidone, de copolyvidone et de monolaurate de sorbitane.

10. Solution pharmaceutique orale selon l'une quelconque des revendications 1 à 9, dans laquelle le vecteur liquide pharmaceutiquement acceptable comprend de l'eau.

11. Solution pharmaceutique orale selon l'une quelconque des revendications 1 à 10, pour son utilisation dans un procédé de traitement dans lequel la solution est administrée par voie orale sans être d'abord diluée avec un vecteur liquide.
